# EUROPEAN PATENT APPLICATION

(11) **EP 4 756 830 A1**
(43) Date of publication of application: **10.06.2026**
(21) Application number: 24217416.7
(22) Date of filing: 04.12.2024
(51) Int. Cl.: G16H 40/40, G16H 30/20, G16H 40/63, G16H 40/67, G16H 50/30, G16H 50/70, G16H 70/20, H05G 1/00, A61B 6/00

(54) **CHARACTERIZING AN EXPECTED RESULT OF AN INTENDED USE OF A MEDICAL APPARATUS AND/OR AN EFFECT OF THE INTENDED USE ON THE MEDICAL APPARATUS**

(71) Applicant: Siemens Healthineers AG, 91301 Forchheim (DE)
(72) Inventor: Baruth, Oliver, 91074 Herzogenaurach (DE); Subrahmanyam, Janapati, 522002 Guntur (IN)
(74) Representative: Siemens Healthineers Patent Attorneys

(57) **Abstract**

For characterizing an expected result of an intended use of a medical apparatus (2) and/or an effect of the intended use on the medical apparatus (2), historic operating data (22) is received containing respective context data (23) for a plurality of historic uses according to an operation profile (18), which specifies values for a plurality of operating parameters. Further context data (24) is determined based on the context data (23). Modified values (25) for a parameter subset of the operating parameters are received. Model output data (27) comprising a modified operation profile (28) is generated by applying a model (32) for the medical apparatus (2) to model input data (26), which comprises the further context data (24) and the modified values (25). The model output data (27) comprises characterizing data (29, 30) regarding the expected result and/or the effect of the intended use.

## Description

The present invention is directed to a computer-implemented method for characterizing an expected result of an intended use of a medical apparatus and/or an effect of the intended use on the medical apparatus, to a corresponding data processing system and a corresponding computer program product.

The operation of a medical apparatus, for example a medical imaging apparatus or a robotic device for endovascular or surgical interventions, is often based on a large number of operating parameters. Changes in a single or few operating parameters may, on the one hand, make it necessary to also modify other operating parameters, for example in order to ensure a desired quality or other property of the result of the operation. On the other hand, such changes in a single or few operating parameters may also have an effect on the medical apparatus itself, for example its expected lifetime or degradation. Since the relation of changing operating parameters and an expected result of an intended use of the medical apparatus or an effect on the medical apparatus itself may be highly complex, a user cannot reliably predict these effects. This may lead to unwanted effects on the result of the intended use and/or on the medical apparatus. Furthermore, it may require multiple iterations of modifying parameters, using the medical apparatus with the modified parameters, and evaluating the result.

For example, assuming the medical apparatus is an X-ray based medical imaging device, increasing an X-ray dose may reduce noise in the resulting X-ray images but, on the other hand, it may also reduce the lifetime of the X-ray tube.

The publication S. Agostinelli et al.: "Geant4-a simulation toolkit", Nuclear Instruments and Methods in Physics Research Section A: Accelerators, Spectrometers, Detectors and Associated Equipment, 506 (3): 250 describes Geant4, a platform for simulating the passage of particles through matter using Monte-Carlo methods.

The publications I. Cunningham et al: "A spatial-frequency dependent quantum accounting diagram and detective quantum efficiency model of signal and noise propagation in cascaded imaging systems.", 1994, Med. Phys., 21: 417-427 and J. Siewerdsen et. al: "Empirical and theoretical investigation of the noise performance of indirect detection, active matrix flat-panel imagers (AMFPIs) for diagnostic radiology.", 1997, Med Phys., 24(1), 71-89, describe analytical models of imaging systems.

It is an objective of the present invention to provide a possibility for automatically and reliably characterizing an expected result of an intended use of a medical apparatus and/or an effect of the intended use on the medical apparatus in case of a modification of operating parameters of the medical apparatus, which does not require to actually use the medical apparatus according to the modified operating parameters.

This objective is achieved by the subject matter of the independent claim. Further implementations and preferred embodiments are subject matter of the dependent claims.

The invention is based on the idea to provide context data specifying a context of a plurality of historic uses of the medical apparatus and to use a model of the medical apparatus to predict characterizing data, which characterizes the expected result of the intended use and/or the effect of the intended use on the medical apparatus, based on the context data and the modified operating parameters.

According to an aspect of the invention, a computer-implemented method for characterizing an expected result of an intended use of a medical apparatus and/or for characterizing an effect of the intended use on the medical apparatus is provided. Therein, historic operating data is received, which contains, for each of a plurality of historic uses of the medical apparatus according to a predefined operation profile, respective context data specifying a context of the respective historic use. Further context data specifying a further context of the intended use of the medical apparatus is determined based on the context data of the plurality of historic uses. The operation profile specifies respective values for a plurality of operating parameters of the medical apparatus. Modified values for a parameter subset of the plurality of operating parameters are received. Model output data, which comprises a modified operation profile for the intended use, is generated by applying a predetermined model for the medical apparatus to model input data. The model input data comprises the further context data and the modified values for the parameter subset. The model output data comprises characterizing data, which characterizes the expected result of the intended use and/or the effect of the intended use on the medical apparatus. Alternatively, the characterizing data, which characterizes the expected result of the intended use and/or the effect of the intended use on the medical apparatus, is determined based on the model output data.

Unless stated otherwise, all steps of the computer-implemented method may be performed by a data processing system, which comprises at least one data processing device. In particular, the at least one data processing device is configured or adapted to perform the steps of the computer-implemented method. For this purpose, the at least one data processing device may for example store a computer program comprising instructions which, when executed by the at least one data processing device, cause the at least one data processing device to execute the computer-implemented method. The expressions "data processing system" and "at least one data processing device" may be used interchangeably, here and in the following. This holds also for respective expressions derived therefrom.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

From each implementation of the computer-implemented method, a respective implementation of a method, which is not purely computer-implemented, is obtained by including respective steps of generating the historic operating data by using the medical apparatus according to the predefined operation profile for the plurality of historic use.

The context data may for example comprise or consist of data or parameters, which cannot or shall not be modified freely but are predefined by the respective use case. This may include parameters of a patient, such as a water equivalent value in case of an X-ray based medical imaging device, but it may also include further operating parameters of the medical apparatus, which are, in particular, not part of the plurality of operating parameters, for example a detector zoom factor of an X-ray detector.

The plurality of operating parameters may for example comprise or consist of parameters, which may be modified freely or at least within certain predefined limits, for example a tube voltage or a focal spot size of an X-ray source.

The further context data corresponds to the context of the intended use. To determine the further context data, the context data of the plurality of historic uses may be evaluated, for example statistically. To this end, it may for example be assumed that the context of the intended use is identical to the context of the plurality of historic uses or the context of the plurality of historic uses on average corresponds to the context of the intended use. The rationale behind this is that it can be expected that the patient statistics and the statistics of use cases will not significantly differ between the plurality of historic uses and future uses, which are modelled by the intended use. In particular, the intended use is an intended use according to the modified operation profile and according to the further context data or, in other words, the further context specified by the further context data.

The parameter subset comprises one or more operating parameters of the plurality of operating parameters. The modified values may for example be received based on a user input. The modified operation profile is for example identical to the operation profile except for the modified operating parameters.

The actual content of the characterizing data depends on the type of the medical apparatus. The characterizing data may for example comprise a measure characterizing an expected lifetime of the medical apparatus or components of it or a measure characterizing a degradation or wear of the medical apparatus or components of it. In case of an imaging system, the characterizing data may for example comprise an image quality measure and/or a dose applied to the patient or staff.

By means of the computer-implemented method according to the invention, the user is enabled to assess the expected result of the intended use and/or the effect of the intended use on the medical apparatus, which is expected to result from the modification of the operating parameter subset. The user may then decide, based on the characterizing data, whether the medical apparatus shall be used in according to the modified operation profile or not. In some embodiments, the characterizing data may also be evaluated automatically by a computer algorithm to decide whether the medical apparatus shall be used in according to the modified operation profile or not. If not, the computer-implemented method may for example be repeated with another set of modified values for the parameter subset or another parameter subset of the plurality of operating parameters. Consequently, a trial-and-error approach of actually using the modified operation profile, evaluating the actual results, adapting the operation profile again and so forth may be avoided. Thus, by means of the computer-implemented method according to the invention, an optimal modification of the operation profile may be achieved in shorter time and/or without risking unwanted effects on the medical apparatus and/or a patient or staff.

For example, the medical apparatus may comprise or consist of a robotic device for surgical or endovascular interventions. The medical apparatus may also comprise or consist of another device for medical treatment and/or diagnostics, for example a high-intensity ultrasonic, HIFU, device, a device for radiotherapy, et cetera. The medical apparatus may also comprise or consist of a medical imaging device, such as an X-ray based medical imaging device, a magnetic resonance imaging device, an ultrasonic imaging device, a positron emission tomography device, et cetera. The medical apparatus may also comprise or consist of a self-driving or autonomous medical device, such as a self-driving C-arm device, et cetera.

According to several embodiments, the medical apparatus comprises or consists of an X-ray based medical imaging device.

In particular, the medical apparatus comprises an X-ray source, which comprises an X-ray tube, and an X-ray detector, as well as a control system for controlling the X-ray source and/or the X-ray detector and/or for receiving acquired data from the X-ray detector.

The X-ray based medical imaging device may for example be a device for monoplane or biplane or multiplane X-ray imaging, for example a C-arm device, or a computed tomography, CT, device, or a cone-beam CT device or an X-ray tomosynthesis device, et cetera.

In such embodiments, the computer-implemented method is particularly beneficial since the achievable image quality, the X-ray dose applied to the patient and/or staff and the lifetime or degradation of the X-ray tube are in a delicate interaction with each other in the sense that a modification of a single operating parameter or few operating parameters usually affects all of them.

In embodiments where the medical apparatus comprises or consists of an X-ray based medical imaging device, the context data may for example comprise a water equivalent value, also denoted as water equivalent diameter or as simply water value, of an object, in particular patient, imaged by the respective historic use.

The water equivalent value has a significant influence for example on the relation between X-ray dose emitted by the X-ray source and the patient X-ray dose, also denoted as skin dose. Thus, taking the water equivalent value into account as a part of the context data allows for a particularly accurate and reliable prediction of the characterizing data.

For example, in case the medical apparatus comprises or consists of an X-ray based medical imaging device, the context data may comprise a detector zoom factor and/or a scene length used during the respective historic use.

The zoom factor corresponds to a predefined part of the X-ray detector used for acquiring the imaging data. By taking the detector zoom factor into account as a part of the context data, the accuracy and reliability of the prediction of the characterizing data are increased.

For example, in case the medical apparatus comprises or consists of an X-ray based medical imaging device, the context data may comprise a filter parameter of an X-ray filter used during the respective historic use.

The filter parameter may for example indicate whether an X-ray filter, for example a copper filter or another metal filter, was used in the respective historic use. The filter parameter may also correspond to a filter thickness of the filter.

The filter parameter has a significant influence for example on the relation between X-ray dose emitted by the X-ray source and the patient X-ray dose as well as on the image quality. Thus, taking the filter parameter value into account as a part of the context data allows for a particularly accurate and reliable prediction of the characterizing data.

For example, in case the medical apparatus comprises or consists of an X-ray based medical imaging device, the context data may comprise a position of an X-ray collimator during the respective historic use.

By taking the position of the X-ray collimator into account as a part of the context data, the accuracy and reliability of the prediction of the characterizing data are increased.

For example, in case the medical apparatus comprises or consists of an X-ray based medical imaging device, the context data may comprise a position of an X-ray source and/or an X-ray detector of the medical apparatus with respect to the object imaged by the respective historic use.

In case of a C-arm device, the position of the X-ray source and/or the X-ray detector may for example be given in terms of an angulation of the C-arm. By taking the position of the X-ray source and/or the X-ray detector into account as a part of the context data, the accuracy and reliability of the prediction of the characterizing data are increased.

According to several embodiments, the plurality of operating parameters comprises at least one operating parameter of the X-ray source, for example the X-ray tube, of the medical apparatus.

The plurality of operating parameters may, for example, comprise a tube voltage of the X-ray source, in particular the X-ray tube, and/or a tube current of the X-ray source, in particular the X-ray tube. The plurality of operating parameters may, for example, comprise a heating current of the X-ray source, in particular the X-ray tube, and/or an X-ray pulse time and/or a focal spot size of the X-ray source.

Said operating parameters of the X-ray source have a significant impact on both the resulting image quality and the applied dose. Said operating parameters of the X-ray source also have a significant impact on the degradation of the X-ray source, for example the X-ray tube, in particular a filament, also denoted as emitter, of the X-ray tube. Thus, by taking into account said operating parameters, the modified operation profile may be determined in a comprehensive way and the accuracy and reliability of the prediction of the characterizing data are increased.

According to several embodiments, the characterizing data, in particular a part of the characterizing data characterizing the expected result of the intended use, comprises an expected image quality measure, for example an expected contrast-to-noise ratio, CNR.

The expected image quality is a particularly important result when using a medical imaging device, for example an X-ray based medical imaging device. Furthermore, the expected image quality is significantly affected by the operating parameters of the medical apparatus, for example of the X-ray source. Thus, the computer-implemented method according to the invention is particularly beneficial in such embodiments.

According to several embodiments, the characterizing data, in particular a part of the characterizing data characterizing the expected result of the intended use, comprises an expected patient X-ray dose and/or an expected staff X-ray dose and/or an expected air kerma and/or an expected dose area product.

The expected patient X-ray dose, the expected staff X-ray dose, the expected air kerma and the expected dose area product are particularly important results when using an X-ray based medical imaging device. Furthermore, said quantities may be subject to regulatory limitations. Thus, the computer-implemented method according to the invention is particularly beneficial in such embodiments.

According to several embodiments, the characterizing data, in particular a part of the characterizing data characterizing the expected result of the intended use, comprises a measure characterizing an expected lifetime of the medical apparatus or of a component of the medical apparatus, and/or a measure characterizing an expected change of the lifetime of the medical apparatus or of the component of the medical apparatus.

According to several embodiments, the characterizing data, in particular a part of the characterizing data characterizing the expected result of the intended use, comprises a measure characterizing an expected lifetime of the X-ray source of the medical apparatus or a component of the X-ray source, for example the X-ray tube or a filament of the X-ray tube. Alternatively or in addition, the characterizing data comprises a measure characterizing an expected change of the lifetime of the X-ray source or of the component of the X-ray source.

The measure characterizing the expected lifetime may for example comprise an estimated lifetime at predefined conditions, for example conditions according to the further context. The lifetime itself may be defined, for example, by comparing one or more predefined properties of the X-ray source or the component of the X-ray source with one or more respective thresholds. The measure characterizing the expected change of the lifetime may for example be a corresponding lifetime reduction, in particular when comparing an operation according to the further context and the operation profile used for the historic uses with an operation according to the further context and the modified operation profile.

To determine the measure characterizing the expected lifetime and/or the measure characterizing the expected change of the lifetime, known lifetime models for X-ray sources, in particular X-ray tubes, may be used. The lifetime model may for example take the modified operation profile and the further context data as inputs and deliver the measure characterizing the expected lifetime and/or the measure characterizing the expected change of the lifetime as an output.

For example, the rate of tungsten evaporation of an emitter of an X-ray tube, given for example in units of µm/h, may be measured. This rate depends on the temperature of the emitter. By adding up the evaporated tungsten, one can estimate when the emitter has become so thin that it fails. The model may be validated by respective measurements.

With regard to the anode side, one may for example calculate the temperature at the focal point. Excessive temperatures may cause melting or cracks. Vaporized material may also cause short circuits in the emitter, also denoted as arcing.

Apart from effects on the image quality and X-ray dose at patient or staff level, the effect of the modification of the operating parameters on the lifetime of the X-ray source is of particular relevance. The computer-implemented method according to the invention allows for an immediate and proactive evaluation of the expected effect on the lifetime in such embodiments.

According to several embodiments, the characterizing data, in particular a part of the characterizing data characterizing the expected result of the intended use, comprises a measure characterizing a degree of wear of the component of the medical apparatus.

The component of the medical apparatus may for example be a movable component. The wear may therefore correspond to a mechanical wear of the moveable component.

For example, the medical apparatus may comprise the movable component and a drive system, which is configured to drive the movable component and the characterizing data may comprise a measure characterizing a degree of wear of the movable component and/or the drive system.

For example, the medical apparatus may comprise a C-arm device and the moveable component is a C-arm of the C-arm device. Alternatively or in addition, the medical apparatus may comprise a robotic device for surgical or endovascular interventions and/or a self-driving medical imaging device, for example a self-driving C-arm device.

The computer-implemented method according to the invention allows for an immediate and proactive evaluation of the expected effect of the modification of the operating parameters on the wear in such embodiments.

According to several embodiments, the medical apparatus is configured to emit an ionizing radiation and the model input data comprises a regulatory limitation regarding a dose, for example the patient X-ray dose or the staff X-ray dose, the air kerma and/or the dose area product, of the ionizing radiation.

The regulatory limitation may for example correspond to a legal obligation to keep the patient X-ray dose, the staff X-ray dose, the air kerma or dose area product below a certain predefined limit. Such limitations restrict the possible modifications of the parameter subset but also the solution space for the model when determining the modified operation profile. Thus, such embodiments avoid additional iterations when trying to find a suitable modified operation profile and increase the reliability of the method.

According to several embodiments, the model input data comprises a technical limitation for the medical apparatus regarding the plurality of operating parameters.

The technical limitation may for example correspond to a predefined allowed or preferred operating range regarding one or more of the plurality of operating parameters. In case of an X-ray imaging device, this may for example be a technical limitation for the X-ray source, in particular the X-ray tube, such as a maximum tube current, a maximum tube voltage, et cetera.

Such limitations restrict the possible modifications of the parameter subset but also the solution space for the model when determining the modified operation profile. Thus, such embodiments avoid additional iterations when trying to find a suitable modified operation profile and increase the reliability of the method.

According to several embodiments, the model for the medical apparatus is generated based on a Monte Carlo simulation of the medical apparatus.

In this way, a reliable and accurate prediction of the modified operation profile and/or the characterizing data, in particular in view of the potentially highly dimensional parameter space, is achieved. The results of the Monte Carlo simulation may for example be provided in terms of one or more lookup tables or the like, which associate a large number of possible combinations of values for the plurality of operating parameters and the further context data with the modified operation profile and/or the characterizing data.

The Monte Carlo simulation may for example be based on Geant4 or similar approaches.

Alternatively or in addition to the Monte Carlo simulation, an analytical model may be used for the model of the medical apparatus, for example an analytical model based on the formalism of Cunningham et al. mentioned above.

According to a further aspect of the invention, a method for using a medical apparatus is provided. Therein, a computer-implemented method according to the invention is carried out and the medical apparatus is used in a further use according to the modified operation profile.

According to a further aspect of the invention, a data processing system that is configured to carry out a computer-implemented method according to the invention is provided.

In the present disclosure, the terms "data processing system" and "at least one data processing device" can be used interchangeably. In particular, a data processing device can be understood to mean a data processing device that contains a processing circuit. The data processing device can therefore, in particular, process data for the purpose of performing computing operations. This may also include operations for performing indexed access to a data structure, for example a look-up table, LUT, as well as a data processing method implemented in hardware.

The data processing device may include, in particular, one or more computers, one or more microcontrollers and/or one or more integrated circuits, for example one or more application-specific integrated circuits, ASIC, one or more field-programmable gate arrays, FPGA, and/or one or more systems-on-a-chip, SoC. The data processing device may also include one or more processors, for example one or more microprocessors, one or more central processing units, CPUs, one or more graphics processing units, GPUs, and/or one or more signal processors, in particular one or more digital signal processors, DSPs. The data processing device may also include a physical or virtual network of computers or other of the mentioned units.

In various embodiments, the data processing device includes one or more hardware and/or software interfaces and/or one or more storage units.

A storage unit may be a volatile data memory, for example a dynamic random access memory, DRAM, or a static random access memory, SRAM, or a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, a system is provided, which comprises a data processing system according to the invention and the medical imaging apparatus.

According to a further aspect of the invention, a computer program comprising instructions is provided. When the instructions are executed by a data processing system, the instructions cause the data processing system to carry out a computer-implemented method according to the invention.

The instructions may be provided as program code, for example. The program code can for example be provided as binary code or assembler and/or as source code of a programming language, for example C, and/or as program script, for example Python.

According to a further aspect of the invention, a computer-readable storage medium, in particular a tangible and/or non-transient computer readable storage medium, storing a computer program according to the invention is provided.

The computer program and the computer-readable storage medium are respective computer program products comprising the instructions.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,
- FIG 1: shows a schematic block diagram of an exemplary embodiment of a system according to the invention;
- FIG 2: shows a schematic flow diagram of an exemplary embodiment of a computer-implemented method according to the invention;
- FIG 3: shows a schematic flow diagram of a further exemplary embodiment of a computer-implemented method according to the invention; and
- FIG 4: shows a schematic flow diagram of a further exemplary embodiment of a computer-implemented method according to the invention.

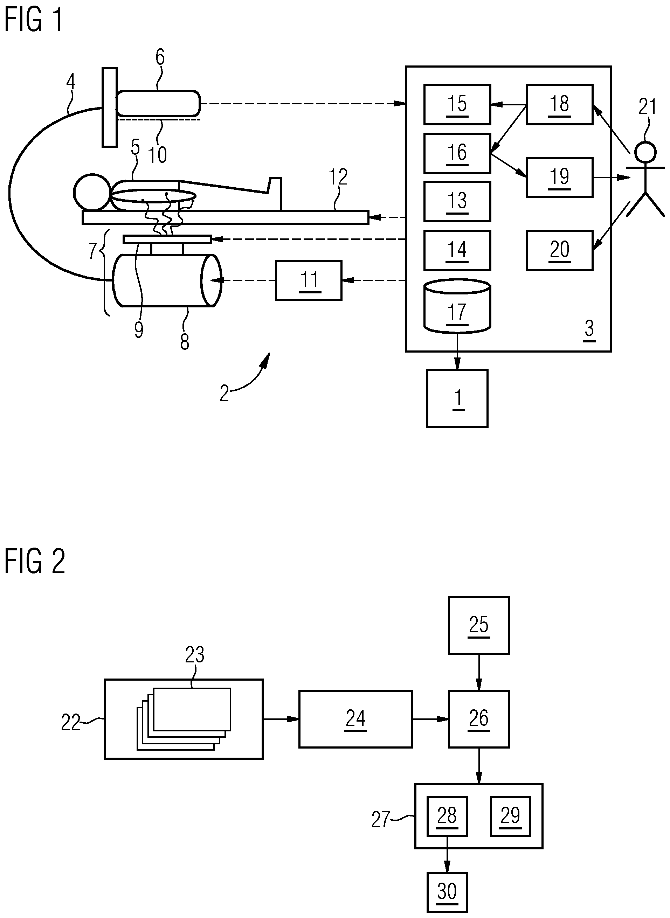

FIG 1 shows schematically an exemplary embodiment of a system according to the invention, the system comprising a medical apparatus 2 and a data processing system 1 according to the invention, which is configured to carry out a computer-implemented method according to the invention.

As an example, the medical apparatus 2 is shown as a C-arm X-ray device with an X-ray source 7 and an X-ray detector 6 mounted on a C-arm 4. The X-ray source 7 may for example comprise an X-ray tube 8 and an X-ray collimator 9. For example, an anti-scattering grid 10 may be arranged next to the X-ray detector 6. A patient 5 may be placed on a patient table 12 of the medical apparatus 2. The following explanations can be applied analogously to other medical apparatus 2.

The medical apparatus 2 comprises, for example a control system 3 with an acquisition controller 13 for controlling the X-ray source 7, for example via a generator 11, and the X-ray detector 6. A movement controller 14 of the control system 3 is provided for controlling a movement of the C-arm 4. The control system 3 also comprises an image acquisition unit 15, which is coupled to the X-ray detector 6 to receive acquired data. An image processing unit 16 of the control system 3 may process the acquired data and displays resulting images on a monitor 19. A user 21 of the medical apparatus 2 may interact with the control system 3 via a control interface 20 of the control system 3. For example, the user 21 may select an operation profile 18, for example in form of a so-called organ program, for the medical apparatus 2. The operation profile 18 specifies respective values for a plurality of operating parameters of the medical apparatus 2.

FIG 2 shows a schematic flow diagram of an exemplary embodiment of a computer-implemented method according to the invention for characterizing an expected result of an intended use of the medical apparatus 2 and/or an effect of the intended use on the medical apparatus 2, which may be carried out by a data processing system 1 of the system of FIG 1.

Therein, historic operating data 22 is received, which contains, for each of a plurality of historic uses of the medical apparatus 2 according to a predefined operation profile 18, respective context data 23 specifying a context of the respective historic use. The historic operating data 22 is for example stored on a storage device 17 of the control system 3, for example in terms of system logs.

Further context data 24 specifying a further context of the intended use of the medical apparatus 2 is determined based on the context data 23 of the plurality of historic uses. Modified values 25 for a parameter subset of the plurality of operating parameters are received, for example from the user 21. Model output data 27, which comprises a modified operation profile 28 for the intended use, is generated by applying a predetermined model for the medical apparatus 2 to model input data 26, which comprises the further context data 24 and the modified values 25 for the parameter subset. The model output data 27 comprises characterizing data 29, which characterizes the expected result of the intended use and/or the effect of the intended use on the medical apparatus 2. Alternatively or in addition, further characterizing data 30, which characterizes the expected result of the intended use and/or the effect of the intended use on the medical apparatus 2, may be determined based on a further part 28 of the model output data 27.

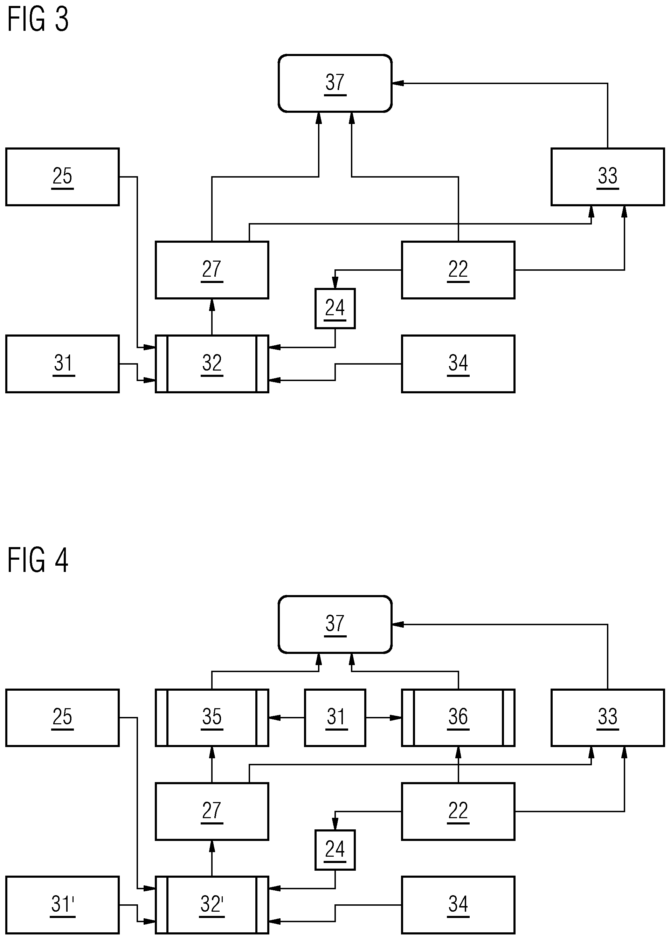

FIG 3 shows a schematic flow diagram of a further exemplary embodiment of a computer-implemented method according to the invention, which is based on the embodiment of FIG 2. The medical apparatus 2 is an X-ray based medical imaging device in this embodiment.

In X-ray-based medical imaging, the more dose invested, the lower is the quantum noise and the better the X-ray image. Good visibility, for example high contrast, low noise, and good resolution, of medical tools or relevant anatomy is necessary for successful interventional therapy. However, there are various clinical disciplines and different procedures and each physician may have a different background and also each anatomy or patient is different. To be able to cope with this, X-ray-based medical imaging systems often offer the possibility to have specific organ programs, OGPs, which can be adapted to match the needs and expectations of the physicians. The plurality of operating parameters may be given by such an OGP and the modified operation profile 28 by a corresponding modified OGP. The parameters of an OGP define, in particular, how the dose regulation for the X-ray source 7 is done in a concrete use case. In the OGP, it may for example be defined which dose control type and which image post-processing is used.

Conventionally, when there is a dose or image quality issue, an image quality or application specialist has to visit the site and to check workflow, images and dose based on local OGP settings, log files and other available data. As a consequence, the specialist may make changes in one or more OGPs. These changes may have positive and/or negative impacts, which will, however, not easily be foreseeable, for example because only a limited number of patients will be treated within the time range of the specialist's visit. For example, the specialist may need to come back at a later time to obtain feedback from the physicians or analyze the data once again when enough patients have been treated with the updated OGP settings. It is difficult to compare various settings at once or to have immediate feedback which impact the OGP changes will have, for example on the lifetime of the X-ray tube 8 and/or staff and/or patient dose over a longer time period. It is possible, for example, that an OGP change which yields only a small reduction of patient skin dose or air kerma but cause large lifetime reduction of the X-ray tube 8, for example due to higher use of a smaller focal spot. In addition, the parameter space is high dimensional, and it is not possible to do many optimization steps at the system itself with real patient traffic.

In respective embodiments, the invention provides a solution, for example in terms of a software solution, to support the specialist's decision finding based on the historic operating data 22.

For example, the characterizing data 27 may characterize the image quality, for example the CNR, of X-ray images and/or a patient skin dose or air kerma, respectively, a dose area product, a lifetime of the X-ray tube 8, an effective patient dose and/or staff dose, et cetera based on the historic operating data 22 and, in particular, the context data 23, such as water equivalent value, scene length, detector zoom and system positions, such as source-to-image distance, SID, angulation of the C-arm 4, position of the collimator 9, et cetera.

The historic operating data 22 may for example comprise the respective radiation related values and system positions, for example stored in structured way so that for each radiation the used OGP, tube voltage, tube current, pulse time, focal spot size, frequency, pre-filter setting, scene length, water equivalent value, et cetera, are available.

For example, the following realistic assumptions may be made: The patient statistics is the same before and after the potential modification. Furthermore, the procedure statistics is also assumed to be the same. That is, the further context data may be determined by statistical evaluation, for example averaging, of the context data 23 of the historic operating data 22. In addition, it may be assumed that the modifications are not too strong, in particular an increase or decrease in image quality does not influence the X-ray statistics significantly, in particular effects of second order may be neglected.

For example, a digital model 32 of the regulation including, for example county specific regulatory dose limitations 34. The model 32 includes for example CNR-tables 31, which are previously determined by means of a Monte Carlo simulation of the medical apparatus 2. The CNR-tables 31 allow it to calculate the optimal operating parameters, for example X-ray acquisition parameters, based on given dose limitations 34, technical limitations regarding the X-ray tube 8, the targeted image quality, for example based on object material, spatial frequency of the object and/or object speed, given detector zoom factor, frequency, tube type, SID, and so forth. Also, other regulation targets like staff dose, effective dose or a combination of them could be used.

In a concrete use case, a specialist identifies an OGP, which results in a too high X-ray patient dose or delivers insufficient image quality. The specialist may provide the modified values 25 for the parameter subset and trigger a simulation step, where the modified parameters will be applied on the related water equivalent values, SIDs, detector zoom factors, scene lengths, and so forth according to the further context data 24. The model output data 27 then includes, on the one hand, the new expected patient skin dose or air kerma, dose area product, staff dose estimations, efficient dose estimation, image quality value, such as CNR, and so forth, and on the other hand all physical regulation parameters like tube voltage, tube current, pulse length filter settings and focal spot size, in other words the modified OGP.

The specialist may then compare the effects of the old OGP with the effects of the modified OGP and decide whether the modification has led to the desired result, for example a reduced patient dose without a significant reduction of the CNR. If this is the case, the modified OGP may be used for a future use. Otherwise, the described steps may be repeated with a different modification. Optionally, also a visualization 37 of the effects of the old OGP versus the effects of the modified OGP may be generated for further assistance.

In some embodiments, a tube lifetime model 33 for the X-ray tube 8 may be provided, which can simulate aging processes of the X-ray tube 8, for example anode and emitter aging, when certain sequences of X-rays are applied. Thus, based on the model output data 27, also the impact of the modifications on lifetime of the X-ray tube 8 may be assessed accordingly.

FIG 4 shows a schematic flow diagram of a further exemplary embodiment of a computer-implemented method according to the invention, which is based on the embodiment of FIG 3.

In this embodiment, a pulse-based dose regulation may be employed instead of the CNR regulation described with respect to FIG 3, wherein the model 32 is replaced by a corresponding model 32' for pulse-based dose regulation. Instead of the CNR-tables 31, the model 32' may use corresponding dose regulation data 31', which may also be determined by means of a Monte Carlo simulation, for example. This may require a higher computing power but also achieve a higher accuracy.

In several embodiments, the steps of the computer-implemented method run locally, meaning that the data processing system 1 is part of the medical apparatus 2, in particular of the control system 3. Alternatively, the steps of the computer-implemented method may run on a general-purpose computer of the specialist or on a cloud computing system or backend computing system. In this case, the historic operating data 22 are for example transferred from the storage device 17 of the control system 3 to the data processing system 1.

In several embodiments, further historic operating data may be used in addition to the historic operating data 22, the further historic operating data corresponding to further historic uses of one or more further medical apparatus according to the operation profile 18, wherein the one or more further medical apparatus are identical or comparable to the medical apparatus 2. Thus, the reliability and accuracy of the approach are further increased.

In several embodiments, a purely computer-based optimization process may be implemented. Instead of or in addition to the manual modification of operating parameters by the specialist, the data processing system 1 could simulate all possible modifications of the plurality of operating parameters to optimize the result according to a given task, for example finding a minimum air kerma, wherein predefined boundary conditions may be used, for example a predefined limitation of a CNR reduction and/or a predefined limitation of the lifetime reduction. In several embodiments, a cloud-based OGP-optimizer could continuously scan incoming x-ray data from multiple medical apparatus. The OGP-optimizer could perform simulations according to a set of known and proven OGP parametrizations on every medical apparatus and for every used OGP. If the OGP-optimizer finds a potential improvement, it could automatically generate a notification to the specialist with a corresponding proposal. To improve the efficiency before the simulation is done, the available data may be analyzed to determine which OGPs are used most often and/or have the biggest impact on the respective targets.

In several embodiments, alternatively or in addition to the lifetime of the X-ray tube 8, other failure channels of the X-ray tube 8, like bearing failures, could be taken into account for the characterizing data 28, 29. In this case, data on the temperature of the X-ray tube 8, system starts and shutdowns, orientation of rotation axis and rotation frequencies, et cetera may be collected and a corresponding lifetime model of the bearing may be set up and used as described for the lifetime model 33.

Independent of the grammatical term usage, individuals with male, female or other gender identities are included within the term.

## Claims

1. Computer-implemented method for characterizing an expected result of an intended use of a medical apparatus (2) and/or an effect of the intended use on the medical apparatus (2), wherein
- historic operating data (22) is received, which contains, for each of a plurality of historic uses of the medical apparatus (2) according to a predefined operation profile (18), respective context data (23) specifying a context of the respective historic use;
- further context data (24) specifying a further context of the intended use of the medical apparatus (2) is determined based on the context data (23) of the plurality of historic uses;
- the operation profile (18) specifies respective values for a plurality of operating parameters of the medical apparatus (2), and modified values (25) for a parameter subset of the plurality of operating parameters are received;
- model output data (27), which comprises a modified operation profile (28) for the intended use, is generated by applying a predetermined model (32) for the medical apparatus (2) to model input data (26), which comprises the further context data (24) and the modified values (25) for the parameter subset;
- the model output data (27) comprises characterizing data (29, 30), which characterizes the expected result of the intended use and/or the effect of the intended use on the medical apparatus (2), or the characterizing data (29, 30) is determined based on the model output data (27).

2. Computer-implemented method according to claim1, wherein the medical apparatus (2) comprises an X-ray based medical imaging device.

3. Computer-implemented method according to claim 2, wherein the context data (23) comprises
- a water equivalent value of an object (5) imaged by the respective historic use; and/or
- a detector zoom factor used during the respective historic use; and/or
- a filter parameter of an X-ray filter used during the respective historic use; and/or
- a position of an X-ray collimator (9) during the respective historic use; and/or
- a position of an X-ray source (7) and/or an X-ray detector (6) of the medical apparatus (2) with respect to the object (5) imaged by the respective historic use.

4. Computer-implemented method according to one of claims 2 or 3, wherein the plurality of operating parameters comprises at least one operating parameter of an X-ray source (7) of the medical apparatus (2).

5. Computer-implemented method according to one of claims 2 to 4, wherein the plurality of operating parameters comprises
- a tube voltage of an X-ray source (7) of the medical apparatus (2); and/or
- a tube current of the X-ray source (7); and/or
- a heating current of the X-ray source (7); and/or
- an X-ray pulse time; and/or
- a focal spot size of the X-ray source (7).

6. Computer-implemented method according to one of claims 2 to 5, wherein the characterizing data (29, 30) comprises
- an expected image quality measure; and/or
- an expected contrast-to-noise ratio, CNR; and/or
- an expected patient X-ray dose and/or an expected staff X-ray dose; and/or
- an expected air kerma and/or an expected dose area product.

7. Computer-implemented method according to one of claims 2 to 6, wherein the characterizing data (29, 30) comprises
- a measure characterizing an expected lifetime of an X-ray source (7) of the medical apparatus (2) or a component of the X-ray source (7); and/or
- a measure characterizing an expected change of the lifetime of the X-ray source (7) or of the component of the X-ray source (7).

8. Computer-implemented method according to one of claims 1 to 6, wherein the characterizing data (29, 30) comprises
- a measure characterizing an expected lifetime of the medical apparatus (2) or of a component of the medical apparatus (2); and/or
- a measure characterizing an expected change of the lifetime of the medical apparatus (2) or of the component of the medical apparatus (2); and/or;
- a measure characterizing a degree of wear of the component of the medical apparatus (2).

9. Computer-implemented method according to one of the preceding claims, wherein the medical apparatus (2) comprises a movable component and a drive system, which is configured to drive the movable component and the characterizing data (29, 30) comprises a measure characterizing a degree of wear of the movable component and/or the drive system.

10. Computer-implemented method according to claim 9, wherein
- the medical apparatus (2) comprises a C-arm device and the moveable component is a C-arm (4) of the C-arm device; or
- the medical apparatus (2) comprises a robotic device for surgical or endovascular interventions; or
- the medical apparatus (2) comprises a self-driving medical imaging device.

11. Computer-implemented method according to one of the preceding claims, wherein
- the medical apparatus (2) is configured to emit an ionizing radiation and the model input data (26) comprises a regulatory limitation (34) regarding a dose of the ionizing radiation; and/or
- the model input data (26) comprises a technical limitation for the medical apparatus (2) regarding the plurality of operating parameters.

12. Computer-implemented method according to one of the preceding claims, wherein determining the further context data (24) comprises a statistical evaluation of the context data (23) of the plurality of historic uses.

13. Computer-implemented method according to one of the preceding claims, wherein the model (32) for the medical apparatus (2) is generated based on a Monte Carlo simulation of the medical apparatus (2) or the model (32) for the medical apparatus (2) is an analytical model of the medical apparatus (2).

14. Data processing system (1), which is configured to carry out a computer-implemented method according to one of the preceding claims.

15. Computer program product comprising instructions, which, when executed by a data processing system (1), cause the data processing system (1) to carry out a computer-implemented method according to one of claims 1 to 13.
